# EUROPEAN PATENT APPLICATION

(11) **EP 4 725 521 A1**
(43) Date of publication of application: **15.04.2026**
(21) Application number: 24823258.9
(22) Date of filing: 03.06.2024
(51) Int. Cl.: A61M 5/162, A61J 1/20

(54) **PUNCTURE NEEDLE, PUNCTURE TOOL, AND PUNCTURE TOOL ASSEMBLY**

(30) Priority: 12.06.2023 JP 2023096104
(71) Applicant: Daiwa Can Company, Chiyoda-ku, Tokyo 100-7009 (JP)
(72) Inventor: ASANO, Toshihiro, Sagamihara-shi, Kanagawa 252-5183 (JP); TAKAMI, Makoto, Sagamihara-shi, Kanagawa 252-5183 (JP)
(74) Representative: AWA Sweden AB
(86) International application number: PCT/JP2024/020222
(87) International publication number: WO 2024/257638

(57) **Abstract**

A puncture needle (14) made of a resin material has a needle part (22) and a groove part (24). The needle part (22) integrally has a trunk part (32) having a path through which a fluid passes, a puncture part (34) provided on the distal end side of the trunk part (32) and having a needle tip at the most distal end, and an intermediate part (36) provided between the trunk part (32) and the puncture part (34). A pair of protruding parts (64) are provided integrally with the intermediate part (36) and are provided integrally with a bottom surface (62) extending from the trunk part (32) toward the puncture part (34), the pair of protruding parts extending from the trunk part (32) toward the puncture part (34) in a direction along an axis (C) of the needle part (22) and also protruding from the bottom surface (62) in a direction intersecting the axis (C) of the needle part (22). In the groove part (24), a channel formed between the bottom surface (62) and the pair of protruding parts (64) communicates with the path of the trunk part (32), and the channel is opened to the outside of the intermediate part (36).

## Description

### FIELD

The present invention relates to a puncture needle made of a resin material, a puncture tool including the puncture needle, and a puncture tool assembly including the puncture tool.

### BACKGROUND

For example, many medical drugs are supplied in the state of being filled into a container sealed with a lid made of an elastic body such as a rubber material. The elastic body used for such a container has various shapes and is made of various materials, and for example, a medical needle made of a synthetic resin is inserted into the elastic body to perform collection, formulation, administration, and the like of a drug.

As described in Jpn. Pat. Appln. KOKAI Publication No. 2014-97102, for example, a puncture needle made of a synthetic resin has been conventionally used to puncture an elastic body of a medicine container filled with a medicine and collect the medicine.

### CITATION LIST

### PATENT LITERATURE

Patent Literature 1: Jpn. Pat. Appln. KOKAI Publication No. 2014-97102

### SUMMARY

### TECHNICAL PROBLEM

When a puncture needle is inserted into an elastic body, a fluid passage of the puncture needle may be blocked by the elastic body depending on the amount of elastic deformation of the elastic body serving as a lid of a container to be used or due to the elastic deformation of the puncture needle itself, or the like. For example, it is assumed that such blockage of a fluid passage of a puncture needle is likely to occur due to an elastic body having a large thickness, an elastic body having a film coating on the inner surface side of a container, or the like.

The present invention has been made to solve such a problem, and an object thereof is to provide a puncture needle made of a resin material that facilitates securing of a fluid passage when an elastic body serving as a lid of a container enclosing a liquid such as a chemical solution is punctured, a puncture tool including the puncture needle, and a puncture tool assembly including the puncture tool.

### SOLUTION TO PROBLEM

A puncture needle made of a resin material, includes: a needle portion, and a groove portion. The needle portion integrally includes: a body portion including a passage configured to pass a fluid through the passage, a puncture portion arranged on a distal side of the body portion and including a needle tip at a most distal end, and an intermediate portion between the body portion and the puncture portion. The groove portion includes: a bottom surface integrally arranged on the intermediate portion and extending from the body portion toward the puncture portion, and a pair of protrusions integrally arranged on the bottom surface, extending from the body portion toward the puncture portion in a direction along an axial center of the needle portion, and protruding from the bottom surface in a direction intersecting the axial center of the needle portion. A channel formed between the bottom surface and the pair of protrusions communicates with the passage of the body portion, and the channel is opened toward an outside of the intermediate portion.

### BRIEF DESCRIPTION OF THE DRAWINGS

FIG. 1 is a schematic perspective view of a puncture tool assembly according to a first embodiment.
FIG. 2 is a schematic perspective view of a front side of a puncture needle of a puncture tool of the puncture tool assembly shown in FIG. 1.
FIG. 3 is a schematic perspective view of a back side of the puncture needle of the puncture tool shown in FIG. 2.
FIG. 4 is a front view of the puncture needle of the puncture tool of the puncture tool assembly shown in FIGS. 1 and 2.
FIG. 5 is a rear view of the puncture needle of the puncture tool shown in FIG. 4.
FIG. 6 is a right-side view of the puncture needle of the puncture tool shown in FIG. 4.
FIG. 7 is a bottom view of the puncture needle of the puncture tool shown in FIG. 6.
FIG. 8 is a cross-sectional view taken along the line VIII-VIII in FIGS. 4 and 5.
FIG. 9 is a schematic cross-sectional view taken along the line IX-IX in FIG. 4.
FIG. 10 is a schematic view showing a state in which a needle portion of the puncture needle is inserted into an elastic body.
FIG. 11A is a schematic view showing a state following FIG. 10, in which the needle portion of the puncture needle is inserted into the elastic body.
FIG. 11B is a schematic cross-sectional view showing the needle portion of the puncture needle shown in FIG. 11A as the cross section shown in FIG. 8.
FIG. 12A is a schematic view showing a state following FIG. 11B, in which the needle portion of the puncture needle is inserted into the elastic body.
FIG. 12B is a schematic cross-sectional view showing the needle portion of the puncture needle shown in FIG. 12A as the cross section shown in FIG. 8.
FIG. 13 is a schematic perspective view showing a state in which a liquid is collected in a channel of a first groove portion with the needle portion of the puncture needle inserted into the elastic body.
FIG. 14A shows a first modification of the cross-sectional view taken along the line IX-IX in FIG. 4.
FIG. 14B shows a second modification of the cross-sectional view taken along the line IX-IX in Fig. 4.
FIG. 14C shows a third modification of the cross-sectional view taken along the line IX-IX in Fig. 4.
FIG. 14D shows a fourth modification of the cross-sectional view taken along the line IX-IX in FIG. 4.
FIG. 14E shows a fifth modification of the cross-sectional view taken along the line IX-IX in FIG. 4.
FIG. 15 is a cross-sectional view of the needle portion shown in FIG. 4, taken along the line VIII-VIII, of a puncture needle according to a second embodiment.
FIG. 16A shows a first modification of the cross-sectional view of the needle portion shown in FIG. 15 taken along the line VIII-VIII.
FIG. 16B shows a second modification of the cross-sectional view of the needle portion shown in FIG. 15 taken along the line VIII-VIII.
FIG. 16C shows a third modification of the cross-sectional view of the needle portion shown in FIG. 15 taken along the line VIII-VIII.
FIG. 17 is a cross-sectional view of the needle portion shown in FIG. 4, taken along the line VIII-VIII, of a puncture needle according to a third embodiment.

### DETAILED DESCRIPTION

Hereinafter, preferred embodiments of the present invention will be described with reference to the drawings.

### (First Embodiment)

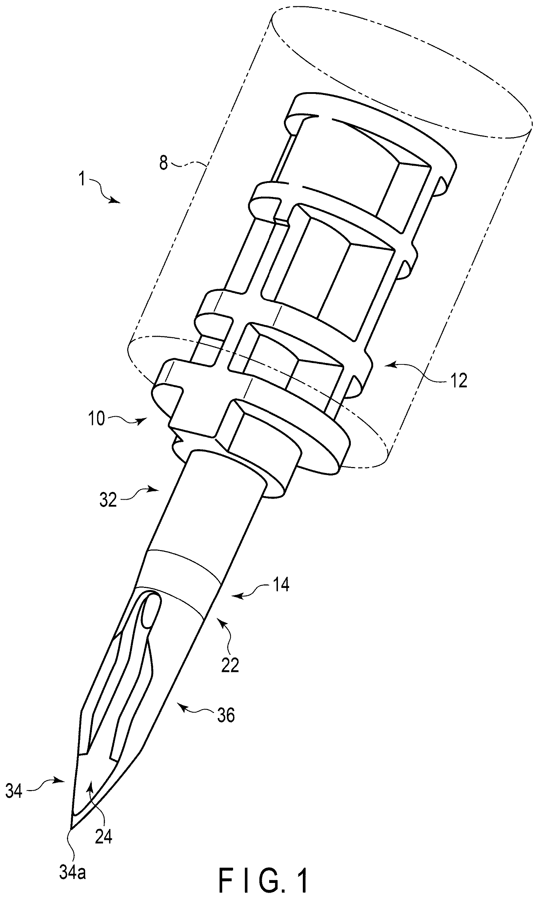

A puncture tool assembly 1 according to a first embodiment will be described with reference to FIGS. 1 to 13.

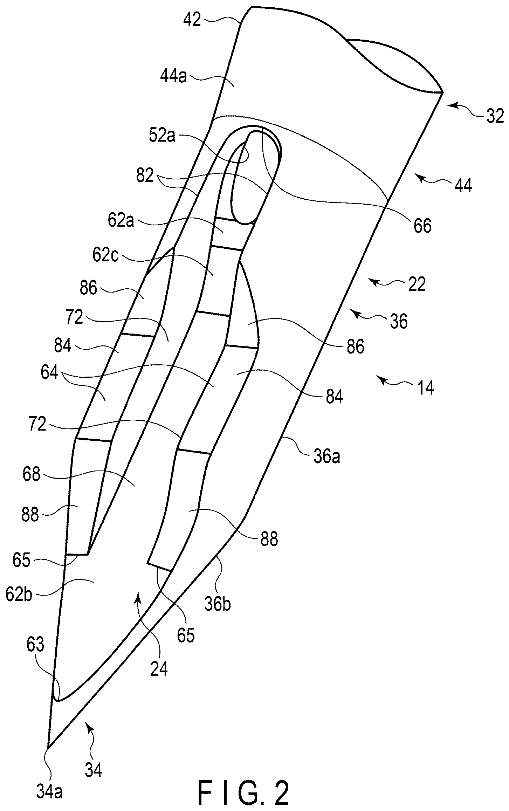

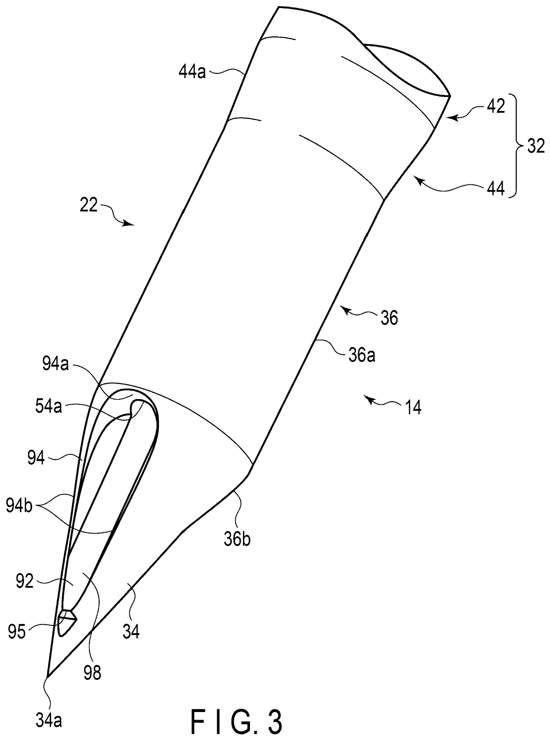

FIG. 1 is a schematic perspective view of the puncture tool assembly 1. FIG. 2 is a schematic perspective view of a front side of a needle portion 22 of a puncture needle 14 of a puncture tool 10 of the puncture tool assembly 1. FIG. 3 is a schematic perspective view of a back side of the needle portion 22 of the puncture needle 14 of the puncture tool 10 of the puncture tool assembly 1.

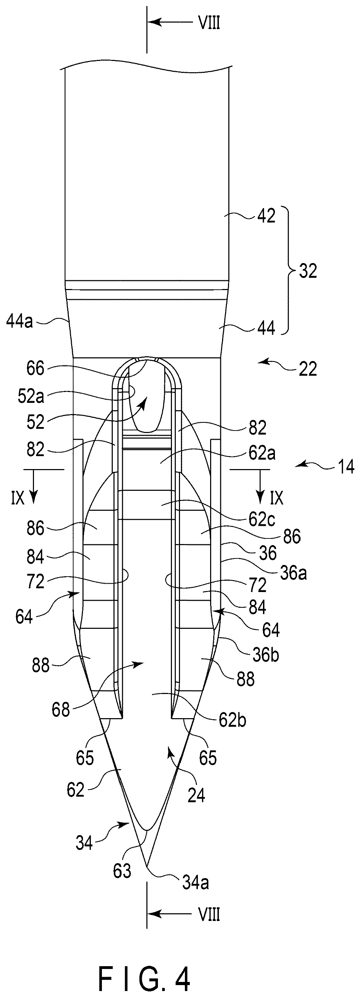

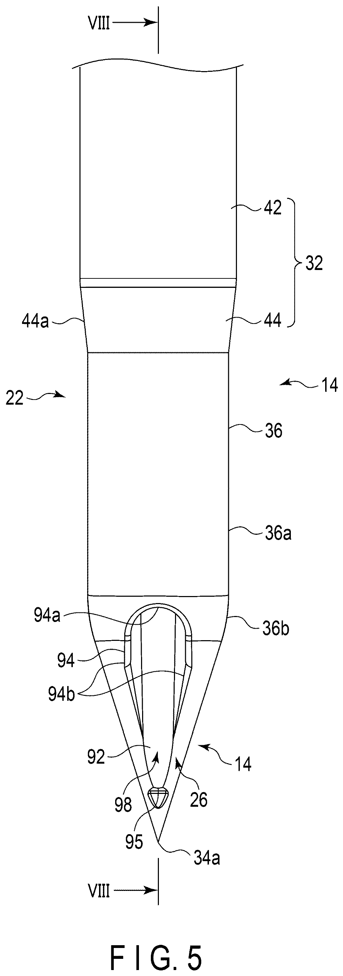

FIG. 4 is a front view of the needle portion 22 of the puncture needle 14 of the puncture tool 10 of the puncture tool assembly 1 shown in FIGS. 1 to 3. That is, a side of the puncture needle 14 on which a later-described first groove portion 24 is exposed to the outside of the puncture needle 14 is defined as the front side of the puncture needle 14. FIG. 5 is a rear view of the needle portion 22 of the puncture needle 14 of the puncture tool 10 shown in FIG. 4. That is, a side of the puncture needle 14 on which a later-described second groove portion 26 is exposed to the outside of the puncture needle 14 is defined as the back side of the puncture needle 14.

When the needle portion 22 of the puncture needle 14 is disposed as shown in FIGS. 4 and 5, the puncture portion 34 side is referred to as "a distal side," and the connection portion 12 side is referred to as "a proximal side." Also, when the needle portion 22 of the puncture needle 14 is disposed as shown in FIG. 4, the right side of the needle portion 22 of the puncture needle 14 shown in FIG. 4 is defined as the right side of the puncture needle 14, and the left side of the needle portion 22 of the puncture needle 14 shown in FIG. 4 is defined as the left side of the puncture needle 14. Thus, when the needle portion 22 of the puncture needle 14 is disposed as shown in FIG. 5, the left side of the needle portion 22 of the puncture needle 14 shown in FIG. 5 is defined as the right side of the puncture needle 14, and the right side of the needle portion 22 of the puncture needle 14 shown in FIG. 5 is defined as the left side of the puncture needle 14.

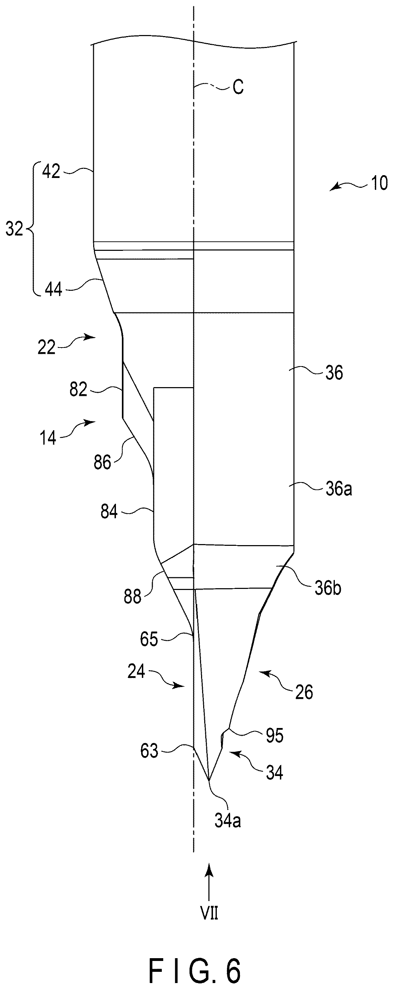

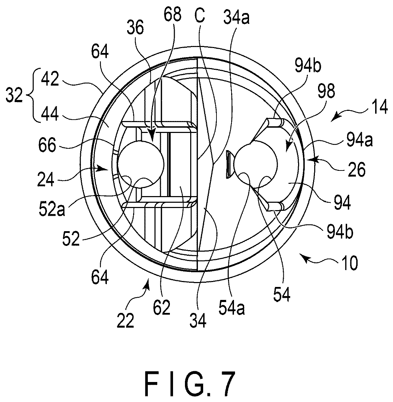

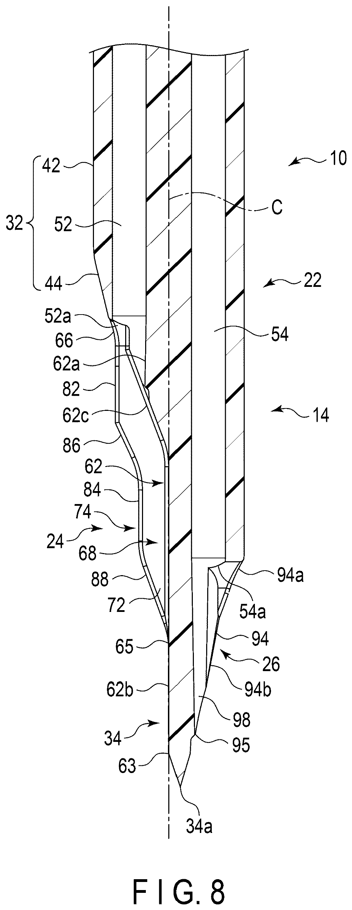

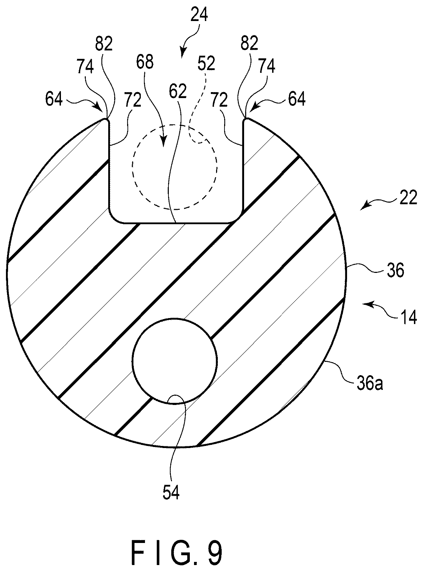

FIG. 6 is a right-side view of the needle portion 22 of the puncture needle 14 of the puncture tool 10 shown in FIG. 4. A left-side view of the needle portion 22 of the puncture needle 14 of the puncture tool 10 is merely a view opposite to the right-side view shown in FIG. 6; thus, the illustration thereof is omitted here. FIG. 7 is a bottom view of the needle portion 22 of the puncture needle 14 of the puncture tool 10 shown in FIG. 6, as viewed from the direction indicated by the arrow VII of the needle portion 22. FIG. 8 is a cross-sectional view taken along the line VIII-VIII in FIGS. 4 and 5. FIG. 9 is a schematic cross-sectional view taken along the line IX-IX in FIG. 4.

The left-right direction (horizontal direction) of the puncture needle 14 of the puncture tool 10 refers to a direction orthogonal to the axial center C of the needle portion 22 when the needle portion 22 of the puncture needle 14 of the puncture tool 10 shown in FIG. 4 is viewed from the front side.

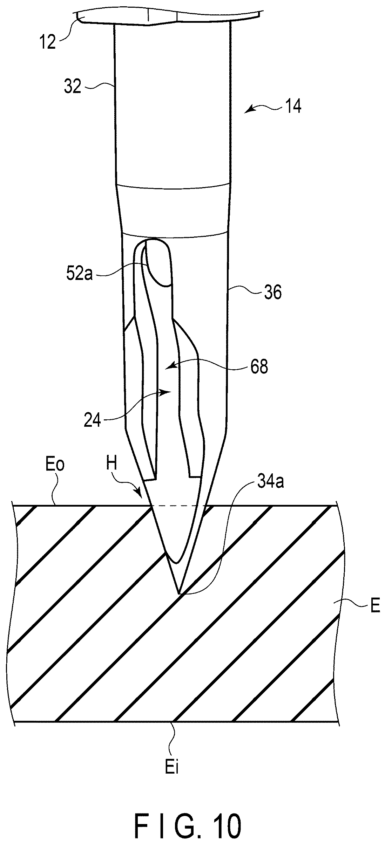

FIGS. 10 to 13 are diagrams explaining a series of operations for inserting the needle portion 22 into an inner surface Ei through an outer surface Eo of an elastic body E of an appropriate container using the puncture tool 10.

For example, butyl rubber, silicone rubber, ethylene tetrafluoroethylene (ETFE), which is a copolymer of tetrafluoroethylene and ethylene, or the like is used as the elastic body E. The hardness of the elastic body E is, for example, from 20 to 55 with respect to Shore A (ISO 7619; ASTM D2240; JIS K 6253). The thickness of the elastic body E is, for example, about 7 mm.

As shown in FIG. 1, the puncture tool assembly 1 includes a connector 8 and the puncture tool 10 made of a resin material and connected to the connector 8.

The connector 8 used may be of various types. An example of the connector 8 is a syringe for supplying a dissolving solution and for sucking and discharging a chemical solution in a container. Another example of the connector 8 is an instrument called an adapter or the like that is attached to a container such as a vial with the puncture needle 14 of the puncture tool 10 inserted into the container. For example, a syringe is connected to a later-described passage 52 of the puncture needle 14 via an adapter.

The puncture needle 14 can puncture the elastic body E shown in FIGS. 10 to 13, and it may also be used without puncturing. Thus, not only a vial but also an ampule, for example, may be used as a container for which the puncture needle 14 is used, and the type of container is not limited. Another example of an object to be punctured with the puncture needle 14 may be an elastic body of a connector of a bag in which a liquid such as a chemical solution or blood is enclosed and which is suspended for use.

The puncture tool 10 includes a connection portion (base portion) 12 and the puncture needle 14 that is extending straight. In the puncture tool 10 according to the present embodiment, the connection portion 12 and the puncture needle 14 are, for example, integrally formed of a resin material. As an example of the resin material, an appropriate thermoplastic resin material such as polyethylene, polypropylene, polyamide, polycarbonate, ABS, PEEK, or the like can be preferably used. The puncture needle 14 according to the present embodiment is formed to have a hardness such that, when a needle tip 34a is inserted into an elastic body E having an appropriate hardness as will be described later, the needle tip 34a is prevented from being easily crushed and can maintain the shape of the puncture needle 14 against the elastic deformation of the elastic body E. The elastic body E is brought into close contact with the outer peripheral surface of the puncture needle 14 with the puncture needle 14 inserted into the elastic body E.

The connection portion 12 is connected to various connectors 8. The connection portion 12 and the puncture needle 14 are preferably formed integrally, but may be formed separately and connected to each other. The connection portion 12 is formed in, for example, a block shape having an outer shape equal to or larger than the diameter of the proximal end of the puncture needle 14.

The puncture needle 14 according to the present embodiment is formed to be bilaterally symmetrical with respect to a plane defined by the line VIII-VIII shown in FIG. 4 and the line VIII-VIII shown in FIG. 5.

As shown in FIGS. 1 to 9, the puncture needle 14 includes the needle portion 22, the first groove portion 24, and the second groove portion 26.

The needle portion 22 includes a body portion 32, a puncture portion 34 on the distal side of the body portion 32, and an intermediate portion 36 between the body portion 32 and the puncture portion 34.

The body portion 32 is formed integrally with the connection portion 12 shown in FIG. 1. The body portion 32 includes a first columnar portion 42 on the connection portion 12 side, and a second columnar portion 44 continuous with the first columnar portion 42 on the distal side of the first columnar portion 42.

For example, the first columnar portion 42 is formed in a columnar shape. For the sake of simplicity of explanation, in the present embodiment, the first columnar portion 42 is formed in a cylindrical shape. The axial center C of the needle portion 22 of the puncture needle 14 coincides with the central axis of the first columnar portion 42.

The first columnar portion 42 is not limited to a columnar shape having a circular cross section, and may be formed in a columnar shape having an N-sided polygonal (N is an integer of 3 or more) cross section, or formed in a columnar shape having an elliptical cross section.

The front side of the outer peripheral surface of the second columnar portion 44 is inclined with respect to the axial center C of the needle portion 22 of the puncture needle 14. In the present embodiment, the back side of the outer peripheral surface of the second columnar portion 44 extends straight, for example, in parallel to the axis center C of the needle portion 22 of the puncture needle 14, as shown in FIG. 6. The second columnar portion 44 is inclined with respect to the axial center C of the needle portion 22 of the puncture needle 14 on the front side of the puncture needle 14 shown in FIG. 1. Thus, the second columnar portion 44 includes an inclined portion 44a in a region closer to the front side than the back side. The inclination direction of the inclined portion 44a is a direction in which the outer diameter of the body portion 32 decreases toward the puncture portion 34 side.

The body portion 32 includes the passage 52 configured to pass a fluid through the passage. The passage 52 is formed as a channel having a circular tubular shape, an elliptical tubular shape, a polygonal tubular shape, or the like extending straight along the axial center C (longitudinal direction) of the needle portion 22 of the puncture needle 14. The passage 52 is used as a passage for a liquid such as a chemical solution. The passage 52 may be used as a passage for a gas such as air.

The puncture portion 34 is arranged on the distal side of the body portion 32 along the axial center C of the needle portion 22. The intermediate portion 36 is arranged between the body portion 32 and the puncture portion 34 along the axial center C of the needle portion 22. The body portion 32 is formed of a resin material integrally with the intermediate portion 36 on the proximal side of the intermediate portion 36. The puncture portion 34 is formed of a resin material integrally with the intermediate portion 36 on the distal side of the intermediate portion 36.

The puncture portion 34 includes the needle tip (pointed end) 34a, which is tapered toward the distal side. As described later, since it is necessary to puncture an elastic body E with the needle tip 34a of the puncture portion 34 first, it is preferable that the needle tip 34a at the most distal end of the puncture portion 34 not include the first groove portion 24 or the second groove portion 26 and be appropriately pointed. It is desirable that the needle tip 34a can form a hole in an elastic body E having an appropriate thickness, such as a rubber plug of a container such as a vial, when the puncture needle 14 is moved in the direction along the axial center C of the needle portion 22 of the puncture needle 14, and penetrate the elastic body E.

The boundary between the puncture portion 34 and the intermediate portion 36 can be set as appropriate. For example, the region from a distal end 63 of a later-described bottom surface 62 of the first groove portion 24 to the needle tip 34a may be where the puncture portion 34 is located, or the region from distal ends 65 of a later-described pair of protrusions 64 of the first groove portion 24 to the needle tip 34a may be where the puncture portion 34 is located. In the present embodiment, the latter case is adopted. Thus, the bottom surface 62 of the first groove portion 24 is not only provided for the intermediate portion 36 but also provided for the puncture portion 34.

The intermediate portion 36 is formed as a frame between the body portion 32 and the puncture portion 34. The intermediate portion 36 includes a first intermediate body (third columnar portion) 36a on the distal side of the second columnar portion 44 of the body portion 32, and a second intermediate body (fourth columnar portion) 36b on the distal side of the first intermediate body 36a.

The first intermediate body 36a of the intermediate portion 36 is formed integrally with the second columnar portion 44 of the body portion 32 as a columnar body in, for example, a cylindrical shape having the same fixed outer diameter as that of the distal end of the second columnar portion 44 of the body portion 32. It is preferable that the back side of the first intermediate body 36a of the intermediate portion 36 extends straight from the back side of the second columnar portion 44 of the body portion 32 in parallel to the axial center C of the needle portion 22.

The second intermediate body 36b of the intermediate portion 36 is formed integrally with the first intermediate body 36a as a frustum body in, for example, a circular truncated conical shape whose outer diameter decreases toward the puncture portion 34 side, and is also formed integrally with the puncture portion 34.

Thus, the intermediate portion 36 is formed as a combination of a columnar body (first intermediate body 36a) on the body portion 32 side and a frustum body (second intermediate body 36b) on the puncture portion 34 side. The outer shape of the intermediate portion 36 may be formed in a columnar shape having an N-sided polygonal (N is an integer of 3 or more) cross section, or formed in a columnar shape having an elliptical cross section.

The first intermediate body 36a of the intermediate portion 36 extends from the distal end of the second columnar portion 44 toward the puncture portion 34 side along the axial center C of the needle portion 22 of the puncture needle 14. The first intermediate body 36a and the second intermediate body 36b of the intermediate portion 36 form the outer peripheral surface of the needle portion 22 extending from the distal end of the second columnar portion 44 of the body portion 32 toward the puncture portion 34 side. The outer peripheral surface of the needle portion 22 formed by the first intermediate body 36a and the second intermediate body 36b of the intermediate portion 36 smoothly continues between the distal end of the second columnar portion 44 and the needle tip 34a of the puncture portion 34. The maximum diameter of the outer peripheral surface of the needle portion 22 formed by the first intermediate body 36a of the intermediate portion 36 is the same as the outer diameter of the distal end of the second columnar portion 44, and there is no region where the outer diameter becomes larger than the outer diameter of the distal end of the second columnar portion 44 toward the puncture portion 34. Thus, from the distal end of the second columnar portion 44 toward the needle tip 34a of the puncture portion 34, the outer diameter of the intermediate portion 36 is either constant or decreasing. The outer diameter gradually decreases from the distal end 65 of the intermediate portion 36 toward the needle tip 34a at the distal end of the puncture portion 34.

The boundary position between the intermediate portion 36 and the puncture portion 34 along the axial center C of the needle portion 22 may be the same or different between the front side and the back side of the needle portion 22. On the front side, the region from the distal ends 65 of the pair of protrusions 64 toward the distal end is where the puncture portion 34 is located, as described above. For example, on the back side, the region from a distal end 95 of the second groove portion 26 toward the distal end is where the puncture portion 34 is located. In this case, the boundary positions between the intermediate portion 36 and the puncture portion 34 on the right side and the left side between the front side and the back side along the axial center C of the needle portion 22 are the positions connecting the distal ends 65 of the pair of protrusions 64 and the distal end 95 of the second groove portion 26.

In the present embodiment, a hole 52a communicating with the outside of the puncture needle 14 is formed in the second columnar portion 44 as a terminal end of the passage 52 on the puncture portion 34 side. The edge of the hole 52a may be formed in a substantially annular shape, for example, in a substantially elliptical shape. In the present embodiment, the edge of the hole 52a is formed, for example, in a substantially elliptical shape when viewed from the front side of the needle portion 22 as shown in FIG. 4, and is formed, for example, in a substantially annular shape when viewed from the lower side of the needle portion 22 as shown in FIG. 7.

A terminal end of the passage 52 on the side opposite to the hole 52a is formed, for example, in the connection portion 12.

In the present embodiment, the body portion 32 and the intermediate portion 36 include a passage 54 different from the passage 52. The passage 54 is formed as a channel having a circular tubular shape, an elliptical tubular shape, a polygonal tubular shape, or the like extending straight along the axial center C (longitudinal direction) of the needle portion 22 of the puncture needle 14. The passage 54 is used as a passage for a gas such as air. The passage 54 may be used as a passage for a liquid such as a chemical solution. The size of the cross sections (open area per unit length) of the passages 52 and 54 may be the same or different.

A hole 54a communicating with the outside of the puncture needle 14 is formed as a terminal end of the passage 54 on the puncture portion 34 side. In the present embodiment, the hole 54a is formed in the intermediate portion 36. The edge of the hole 54a may be formed in a substantially annular shape, for example, in a substantially elliptical shape. In the present embodiment, the edge of the hole 54a is formed, for example, in a substantially annular shape when viewed from the lower side of the needle portion 22 as shown in FIG. 7.

A terminal end of the passage 54 on the side opposite to the hole 54a is formed, for example, in the connection portion 12.

In the present embodiment, the first groove portion 24 is arranged on the front side of the intermediate portion 36 of the needle portion 22. Thus, the first groove portion 24 is formed on the front side of the puncture needle 14. The length of the first groove portion 24 along the axial center C of the needle portion 22 is formed to be a length that penetrates the inner surface Ei of a plate-shaped elastic body E to be punctured from the outer surface Eo side of the elastic body E.

The first groove portion 24 includes the bottom surface 62 continuous with the inner peripheral surface of the passage 52, the pair of protrusions 64 protruding from the bottom surface 62, and a closed end 66 on the body portion 32 side.

The bottom surface 62 is arranged integrally with the intermediate portion 36 and extends along the axial center C (longitudinal direction) of the needle portion 22 from the body portion 32 toward the puncture portion 34. The bottom surface 62 may be formed as a flat surface or a curved surface. In the present embodiment, the bottom surface 62 is formed by combining a flat surface and a curved surface. The body portion 32 side of the bottom surface 62 is formed as a surface (first bottom surface) 62a continuous with the inner peripheral surface of the passage 52. The first bottom surface 62a is formed, for example, as a flat surface. The puncture portion 34 side of the bottom surface 62 is formed as a surface (second bottom surface) 62b extending straight along the axial center C of the needle portion 22 of the puncture needle 14. The second bottom surface 62b is formed, for example, as a flat surface. In the second bottom surface 62b, the side closest to the needle tip 34a of the puncture portion 34 is defined by the distal end 63 of the bottom surface 62. The distal end 63 of the bottom surface 62 is also the distal end of the first groove portion 24. In the second bottom surface 62b, a portion from the distal ends 65 of the pair of protrusions 64 to the distal end 63 of the first groove portion 24 is formed as a substantially isosceles triangular surface having the distal end 63 as a vertex with the outer peripheral surface of the puncture portion 34 as two equal sides. In practice, the distal end 63 of the first groove portion 24 is preferably formed to have a larger angle than the angle of the needle tip 34a of the puncture portion 34.

A third bottom surface 62c as an inclined surface is formed between the first bottom surface 62a and the second bottom surface 62b, the third bottom surface 62c being inclined with respect to the first bottom surface 62a and the second bottom surface 62b, being continuous with the first bottom surface 62a, and being continuous with the second bottom surface 62b.

When the width of the bottom surface 62 is set to be the outer diameter formed by the intermediate portion 36, the bottom surface 62 is preferably formed to be longer than the width of the bottom surface 62 in the direction along the axial center C of the needle portion 22.

When the bottom surface 62 is convexed toward the front side, the height of the bottom surface 62 is formed to be lower than the height of the pair of protrusions 64 in order to secure a channel 68 of the first groove portion 24.

In the present embodiment, the axial center C of the needle portion 22 of the puncture needle 14, that is, the central axis of the first columnar portion 42 of the body portion 32, is along the second bottom surface 62b of the first groove portion 24. As shown in FIGS. 6 and 7, the needle tip 34a is shifted toward the back side of the needle portion 22 with respect to the axial center C of the needle portion 22 of the puncture needle 14. The axial center of the second columnar portion 44 of the body portion 32 is shifted toward the back side of the needle portion 22 with respect to the axial center C of the first columnar portion 42. The needle tip 34a is shifted toward the back side with respect to the axial center of the second columnar portion 44. The needle tip 34a is formed as a substantially conical region that is tapered as the distance from the body portion 32 side increases.

Thus, the bottom surface 62 of the first groove portion 24 according to the present embodiment is arranged in a position along the inner peripheral surface of the passage 52 on the back side of the needle portion 22, and is arranged on the back side of the needle portion 22 (the side opposite to the opening side (the pair of protrusions 64 side) of the first groove portion 24) with respect to the inner peripheral surface of the passage 52 on the back side of the needle portion 22.

The protrusion 64 and the closed end 66 of the first groove portion 24 are formed as an edge portion of a substantially U-shaped slit that extends in parallel to the axial center C on the front side of the needle portion 22 in the intermediate portion 36, opens the puncture portion 34 side of the puncture needle 14, and closes the body portion 32 side.

In the present embodiment, the width between the pair of protrusions 64 orthogonal to the axial center C of the needle portion 22 is formed to be constant in any position along the axial center C of the needle portion 22.

The pair of protrusions 64 extend on the bottom surface 62 along the axial center C (longitudinal direction) of the needle portion 22 from the body portion 32 toward the puncture portion 34, and protrude from the bottom surface 62 in a direction intersecting the axial center C. The pair of protrusions 64 can function as ribs on the front side that suppress the buckling of the puncture needle 14. The protruding directions of the pair of protrusions 64 with respect to the bottom surface 62 are the same direction. In the present embodiment, the protruding direction of the pair of protrusions 64 with respect to the bottom surface 62 is the front side of the needle portion 22 (see FIG. 4). Thus, the pair of protrusions 64 forms, together with the bottom surface 62, the channel (flow passage) 68 that communicates with the passage 52 and the hole (opening edge) 52a, opens from the axial center C of the needle portion 22 to the outer peripheral surface of the intermediate portion 36, and, in the present embodiment, opens toward the front side. Thus, a fluid such as a liquid or a gas can be put into and taken out from the channel 68.

The pair of protrusions 64 are formed such that the protrusion height of the pair of protrusions 64 with respect to the bottom surface 62 gradually decreases from the second columnar portion 44 side toward the puncture portion 34 side. Thus, the pair of protrusions 64 are formed so as to gradually approach the axial center C of the needle portion 22 from the closed end 66 toward the puncture portion 34.

The pair of protrusions 64 each include a wall surface 72 continuous with the bottom surface 62 and a protrusion end (opening edge) 74.

As shown in FIGS. 2, 4, and 9, the wall surfaces 72 of the pair of protrusions 64 are preferably formed as parallel flat surfaces facing each other. The distance between the protrusion ends 74 of the pair of protrusions 64 protruding from the bottom surface 62 is the same as the width of the bottom surface 62 of the first groove portion 24. The distance between the wall surfaces 72 of the pair of protrusions 64 can be set as appropriate. Thus, the bottom surface 62 and the wall surfaces 72 of the pair of protrusions 64 form the channel (flow passage) 68 having a substantially U-shaped cross section intersecting the axial center C of the needle portion 22 of the puncture needle 14, such as a cross section orthogonal to the axial center C of the needle portion 22 of the puncture needle 14.

It is preferable that a part of a region of the protrusion ends 74 of the pair of protrusions 64 from the second columnar portion 44 side toward the puncture portion 34 side include a region parallel to the axial center C of the needle portion 22 of the puncture needle 14. In the present embodiment, the protrusion ends 74 of the pair of protrusions 64 each include a first edge (first parallel portion) 82 and a second edge (second parallel portion) 84 that are preferably parallel to the axial center C of the needle portion 22 of the puncture needle 14 from the second columnar portion 44 side toward the puncture portion 34 side.

In FIGS. 2, 4, and 9, the first edge 82 with a narrow width in the left-right direction is formed as a substantially linear edge along the axial center C on the outer peripheral surface of the intermediate portion 36. The second edge 84 with a width larger than the width of the first edge 82 in the left-right direction is formed as a flat surface parallel to the axial center C of the needle portion 22 and the second bottom surface 62b. The second edge 84 is also preferably formed as a curved surface, instead of a flat surface, such as a surface having an arc shape or an elliptical arc shape protruding toward the front side opposite to the back side. As with the first edge 82, the second edge 84 is also preferably formed as an edge whose surface on the furthest front side is substantially linear.

The protrusion end 74 includes a first inclined portion 86 between the first edge 82 and the second edge 84, the first inclined portion 86 being inclined with respect to the first edge 82 and the second edge 84. The protrusion end 74 includes a second inclined portion 88 between the second edge 84 and the bottom surface 62 on the needle tip 34a side, the second inclined portion 88 being inclined with respect to the second edge 84.

The first inclined portion 86 is formed such that the protruding height with respect to the third bottom surface 62c gradually decreases from the body portion 32 side toward the puncture portion 34 side. The second inclined portion 88 is formed such that the protruding height with respect to the second bottom surface 62b gradually decreases from the body portion 32 side toward the puncture portion 34 side. The position on the most distal side (the distal end of the intermediate portion 36) indicated by the reference numeral 65 in the second inclined portion 88 is continuous with the second bottom surface 62b. The position on the most distal side indicated by the reference numeral 65 in the second inclined portion 88 is the position of the proximal end of the puncture portion 34.

The first edge 82, the second edge 84, the first inclined portion 86, and the second inclined portion 88 form an opening edge of the channel 68.

The second bottom surface 62b of the bottom surface 62 of the first groove portion 24 is formed on the back side of the needle portion 22 with respect to the surface of the passage 52 on the back side in the body portion 32. The body portion 32 side of the protrusion ends 74 of the pair of protrusions 64 is along or substantially along the surface of the passage 52 on the front side. Thus, even if the elastic body E enters the channel 68 between the pair of protrusions 64 from the front side of the needle portion 22, the flow passages of the channel 68 and the passage 52 are secured more reliably by making the flow passage (depth) for a fluid such as a liquid formed by the channel 68 larger than the diameter of the passage 52.

The outer peripheral surface of the puncture portion 34 is arranged on the back side of the edge portions in the left-right direction of the region of the second bottom surface 62b of the first groove portion 24 from the distal ends 65 of the pair of protrusions 64 toward the further distal end.

The closed end 66 is formed near the boundary between the second columnar portion 44 of the body portion 32 and the first intermediate body 36a of the intermediate portion 36. A part of the closed end 66 is formed as a part of the outer peripheral surface of the first intermediate body 36a of the intermediate portion 36, and is formed as a part of the opening edge of the hole 52a of the passage 52.

The distal end of the channel 68 is defined by the distal ends 65 of the pair of protrusions 64, and the proximal end of the channel 68 is defined by the closed end 66. Thus, the channel 68 is formed between the distal ends 65 and the closed end 66 of the pair of protrusions 64.

In the present embodiment, the body portion 32 includes the passage 54 different from the passage 52, and the hole 54a which is formed as a terminal end of the passage 54 on the needle tip 34a side described later and communicates with the outside. The passage 54 is formed as a channel extending straight along the axial direction of the puncture needle 14. The passage 54 is used as a passage for a gas such as air. The hole 54a of the passage 54 is preferably located closer to the needle tip 34a than the hole 52a of the passage 52. The position of the hole 54a of the passage 54 may be adjacent to the position of the hole 52a of the passage 52 with the axial center C of the needle portion 22 of the puncture needle 14 interposed therebetween. The edge of the hole 54a may be formed in a substantially annular shape, for example, in a substantially elliptical shape.

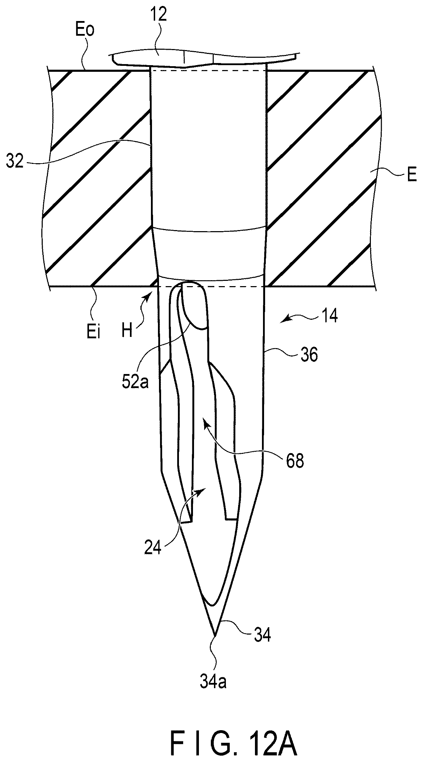

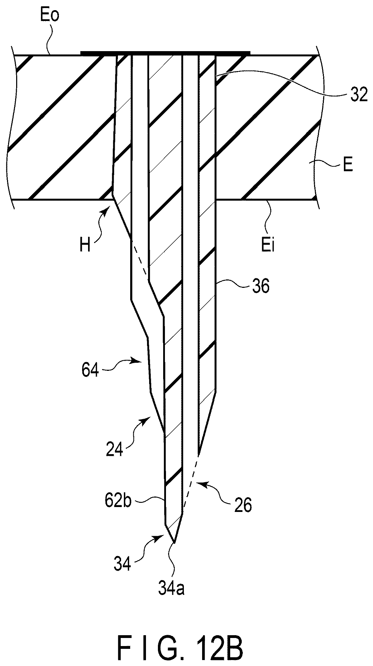

If the distance from the proximal end of the body portion 32 (the distal end of the connection portion 12) to the closed end 66 of the first groove portion 24 is short and the length of the first groove portion 24 is an appropriate length, the channel 68 of the first groove portion 24 is likely to be adjacent to the inner surface Ei of the elastic body E when the puncture needle 14 punctures the elastic body E as shown in FIG. 12A and 12B. Thus, it is assumed that the amount of the residual liquid in a container can be reduced by the action of the puncture needle 14 described later. Depending on the puncture depth of the needle portion 22 with respect to the elastic body E, there is a possibility that the outer surface Eo and the inner surface Ei of the elastic body E are communicated with each other by the first groove portion 24. As the distance from the distal end of the connection portion 12 to the distal end of the first groove portion 24 becomes longer, it is easier to secure a flow passage for a fluid even with a thick elastic body. If the entire length of the first groove portion 24 is too long, there is a possibility that the outer surface Eo and the inner surface Ei of the elastic body E are communicated with each other by the first groove portion 24. Thus, the length between the closed end 66 of the first groove portion 24 and the distal end 63 of the first groove portion 24 is set as appropriate.

In the present embodiment, the second groove portion 26 is arranged on the back side of the intermediate portion 36. Thus, the second groove portion 26 is formed on the back side of the puncture needle 14. The second groove portion 26 is formed as a substantially U-shaped slit extending in parallel to the axial center C, opening the needle tip 34a side of the puncture portion 34 of the puncture needle 14, and closing the body portion 32 side.

As shown in FIGS. 3 and 5, the second groove portion 26 of the puncture portion 34 is formed integrally with the intermediate portion 36 and the puncture portion 34 using a resin material. The second groove portion 26 includes a bottom surface 92 continuous with the inner peripheral surface of the passage 54 and a pair of protrusions 94 protruding from the bottom surface 92.

The bottom surface 92 may be formed as a flat surface or a curved surface. In the present embodiment, the bottom surface 92 is long in the direction along the axial center C of the needle portion 22 and extends in a convexed shape toward the front side. The distal end of the bottom surface 92 is located on the back side with respect to the needle tip 34a. Thus, the bottom surface 62 of the first groove portion 24 and the bottom surface 92 of the second groove portion 26 are adjacent to each other with a shaft including the needle tip 34a parallel to the axial center C interposed therebetween.

The pair of protrusions 94 include a closed end 94a and a pair of edges 94b.

The closed end 94a is formed, for example, near the boundary between the first intermediate body 36a and the second intermediate body 36b of the intermediate portion 36. The closed end 94a is formed as a part of the outer peripheral surface of the second intermediate body 36b of the intermediate portion 36.

The pair of edges 94b are formed as a part of the outer peripheral surface of the intermediate portion 36 and the puncture portion 34. The pair of edges 94b can function as ribs on the back side that suppress the buckling of the puncture needle 14. The pair of edges 94b extend from the second intermediate body 36b of the intermediate portion 36 toward the needle tip 34a of the puncture portion 34, and come closer to each other as the edges 94b approach the needle tip 34a of the puncture portion 34. Thus, the pair of edges 94b are formed such that the width becomes narrower toward the needle tip 34a of the puncture portion 34. Also, the pair of edges 94b approach the axial center C of the needle portion 22 as they go from the second intermediate body 36b of the intermediate portion 36 toward the needle tip 34a of the puncture portion 34. Thus, it is preferable that the pair of edges 94b of the second groove portion 26 not include a portion parallel to the axial center C of the needle portion 22.

The pair of protrusions 94 forms, together with the bottom surface 92, a channel (flow passage) 98 that communicates with the passage 54 and the hole (opening edge) 54a, opens from the axial center C of the needle portion 22 toward the outside of the puncture portion 34 and the intermediate portion 36, and, in the present embodiment, opens toward the back side. Thus, a fluid such as a liquid or a gas can be put into and taken out from the channel 98.

The passages 52 and 54 are preferably formed not only in the puncture needle 14 but also in the connection portion 12 that is formed integrally with the puncture needle 14. The passages 52 and 54 are formed in the connection portion 12 and the puncture needle 14 as through holes parallel to the axial center C of the needle portion 22. That is, the passages 52 and 54 are preferably formed as through holes parallel to the axial center C of the needle portion 22 of the puncture tool 10.

The dimensions of the needle portion 22 of the puncture needle 14 are shown. The following dimensions are examples, and may be changed as appropriate depending on the elastic body E to be punctured, the length and material of the needle portion 22 of the puncture needle 14 to be used, and the like.

The length of the puncture needle 14 is, for example, 20.1 mm. Within this length, the length of the body portion 32 of the needle portion 22 is, for example, 7.6 mm, the length of the intermediate portion 36 is, for example, 8.5 mm, and the length of the puncture portion 34 is, for example, 4 mm.

The outer diameter of the first columnar portion 42 of the body portion 32 is, for example, 4 mm, and the outer diameter of the second columnar portion 44 and the outer diameter of the intermediate portion 36 are, for example, 3.6 mm.

The length of the first groove portion 24 along the axial center C of the needle portion 22 depends on the thickness of the elastic body E, but is preferably 7.5 mm or more.

The length of the pair of protrusions 64 of the first groove portion 24 along the axial center C of the needle portion 22 is, for example, 8.5 mm. Within this length, the length of the first edge (first parallel portion) 82 on the body portion 32 side is, for example, 2.7 mm, and the length of the second edge (second parallel portion) 84 on the puncture portion 34 side is, for example, 2.2 mm. Thus, the length of the first edge 82 on the body portion 32 side is larger than the length of the second edge 84 on the puncture portion 34 side. The length of the first inclined portion 86 along the axial center C of the needle portion 22 between the edges 82 and 84 is, for example, 1.3 mm, and the length of the second inclined portion 88 on the puncture portion 34 side along the axial center C of the needle portion 22 is, for example, 2.2 mm. The inclination angle of the first inclined portion 86 on the body portion 32 side is, for example, 25° with respect to the edges 82 and 84, and the inclination angle of the second inclined portion 88 on the puncture portion 34 side is, for example, 20° with respect to the edges 82 and 84. Thus, the inclination angle of the first inclined portion 86 on the body portion 32 side is larger than the inclination angle of the second inclined portion 88 on the puncture portion 34 side with respect to the edges 82 and 84. Therefore, the inclined portion 88 on the puncture portion 34 side is gentler than the inclined portion 86 on the body portion 32 side.

The distance between the wall surfaces 72 of the pair of protrusions 64 is constant, for example, 1.4 mm from the body portion 32 side to the puncture portion 34 side. That is, the width between the edges 82 of the pair of protrusions 64, the width between the edges 84 of the pair of protrusions 64, the width between the inclined portions 86 of the pair of protrusions 64, and the width between the inclined portions 88 of the pair of protrusions 64 are respectively 1.4 mm. The protruding height of the second edge 84 with respect to the bottom surface 62 of the pair of protrusions 64 is, for example, 0.8 mm, and the protruding height of the first edge 82 is, for example, 1.4 mm.

The diameter of the passages 52 and 54 is, for example, 0.9 mm.

The dimensional relationship between the intermediate portion 36 and the first groove portion 24 is preferably, for example, the diameter of the passage 52 ≤ the width between the wall surfaces 72 of the first groove portion 24 ≤ the width between the edges 82 and 84 < the diameter of the intermediate portion 36.

The dimensions of the puncture needle 14 can be appropriately set depending on the hardness, deformability, and the like of the elastic body E that closes the container. As described above, the hardness of the elastic body E is, for example, from 20 to 55 with respect to Shore A (ISO 7619; ASTM D2240; JIS K 6253). When the hardness of the elastic body E is such a degree, the protruding height (depth) from the bottom surface 62 of the first groove portion 24 to the protrusion ends 74 of the pair of protrusions 64, that is, the depth of the channel 68, is, for example, preferably larger than 0.5 mm, and more preferably 0.8 mm or more. When at least the puncture portion 34 and the intermediate portion 36 of the puncture needle 14 are inserted into the elastic body E, blockage of the channel 68 of the first groove portion 24 due to elastic deformation of the elastic body E is suppressed.

When the width between the protrusion ends 74 of the pair of protrusions 64 is 1.4 mm, the depth of the first groove portion 24 is, for example, 0.5 mm or more. Thus, the depth of the first groove portion 24 is preferably 36% or more of 1.4 mm, which is the width of the first groove portion 24 (the width between the wall surfaces 72 of the pair of protrusions 64). Since the depth of the first groove portion 24 is more preferably 0.8 mm or more, the depth of the first groove portion 24 is more preferably 58% or more of the width of the first groove portion 24. With the elastic body E having the hardness described above, and the depth of the first groove portion 24 being 36% or more of the width of the first groove portion 24, the channel 68 is prevented from being blocked by the elastic body E in a state where the puncture needle 14 punctures the elastic body E.

The axial center C of the needle portion 22 coincides with the central axis (axial center) of the first columnar portion 42, as described above. The axial center of the second columnar portion 44 is eccentric toward the back side by, for example, 0.2 mm from the axial center of the first columnar portion 42. The axial center including the needle tip 34a of the puncture portion 34 is also eccentric toward the back side by, for example, 0.1 mm from the axial center of the second columnar portion 44. Thus, the axial center C of the needle portion 22 is deviated from the axial center including the needle tip 34a.

The operation of the puncture needle 14 according to the present embodiment will be described with reference to FIGS. 10 to 13.

For example, a syringe as the connector 8 for putting air (gas) into a container and collecting a liquid, the above-described adapter, or the like is connected to the connection portion 12 of the puncture tool 10 in advance. Alternatively, the connector 8 for transferring a liquid to another container is connected to the connection portion 12 of the puncture tool 10 in advance.

An example of an object to be punctured with the puncture needle 14 of the puncture tool 10 according to the present embodiment is an elastic body (rubber plug) E of a container (not shown) such as a vial, which is filled with a chemical solution and sealed with a plate-shaped elastic body E made of a rubber material (see FIGS. 10 to 13).

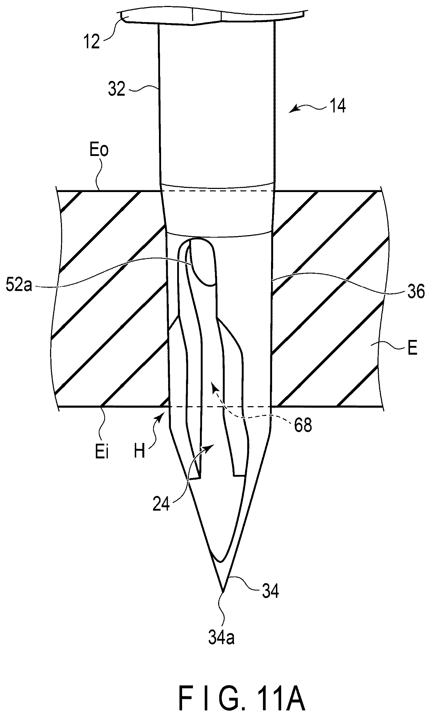

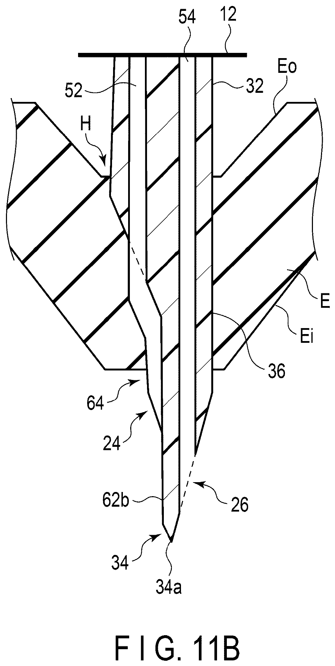

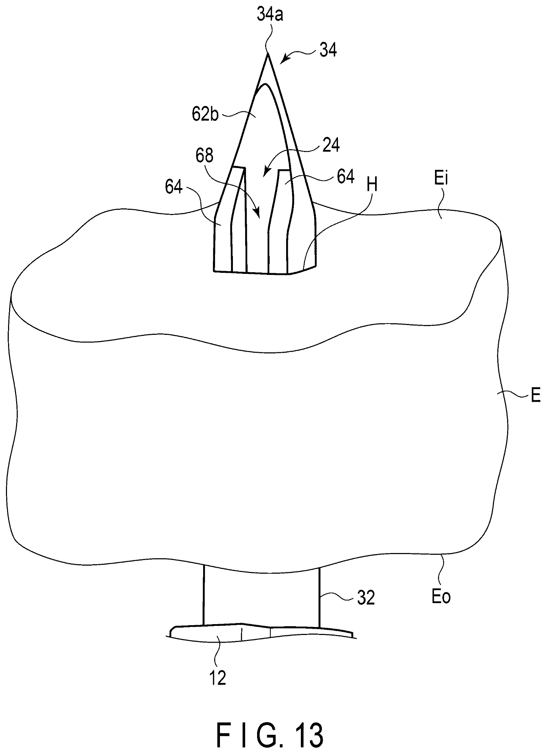

FIGS. 10, 11A, and 12A show a series of diagrams in which a container is punctured with the puncture needle 14 with the outer surface Eo of the elastic body E of the container positioned on the upper side and the inner surface Ei thereof positioned on the lower side. FIG. 11B is a schematic cross-sectional view of the needle portion 22 shown in FIG. 11A, and FIG. 12B is a schematic cross-sectional view of the needle portion 22 shown in FIG. 12A. FIG. 13 is a diagram in which the outer surface Eo of the elastic body E is on the lower side and the inner surface Ei thereof is on the upper side in a state where the elastic body E is punctured by the needle portion 22.

As shown in FIG. 10, a user of the puncture tool 10 such as a doctor or a nurse punctures the outer surface Eo of the elastic body E of the container with the needle tip 34a of the needle portion 22 of the puncture tool 10, thereby forming a puncture hole H of the elastic body E. At this time, the user makes the axial center C of the needle portion 22 orthogonal to the outer surface Eo of the elastic body E, and punctures the elastic body E without rotating the needle portion 22 about the axial center C of the needle portion 22.

As shown in FIGS. 11A and 11B, the user of the puncture tool 10 further inserts the needle portion 22 into the puncture hole H of the elastic body E, and causes the first groove portion 24 to reach the inner surface Ei of the elastic body E. The needle portion 22 of the puncture needle 14 punctures the inner surface Ei of the elastic body E with the needle tip 34a while the outer peripheral surface of the intermediate portion 36, a part of the second bottom surface 62b of the first groove portion 24 on the distal end 63 side, the protrusion ends 74 of the pair of protrusions 64, and the pair of protrusions 94 of the second groove portion 26, in addition to the outer peripheral surface of the puncture portion 34, are in close contact with the elastic body E. Thus, the outer peripheral surface of the puncture portion 34, the outer peripheral surface of the intermediate portion 36, a part of the second bottom surface 62b of the first groove portion 24 on the distal end 63 side, the protrusion ends 74 of the pair of protrusions 64, and the pair of protrusions 94 of the second groove portion 26 push aside the elastic body E, whereby the puncture hole H penetrating the elastic body E is formed.

The body portion 32 also pushes away the elastic body E around the puncture hole H. In the present embodiment, the state is maintained in which the elastic body E around the puncture hole H is pushed away from the outer surface Eo to the inner surface Ei of the elastic body E by the outer peripheral surface of the intermediate portion 36, the protrusion ends 74 of the pair of protrusions 64 of the first groove portion 24, and the outer peripheral surface of the body portion 32. Thus, in the puncture needle 14 that has punctured the elastic body E, the channel 68 of the first groove portion 24 is not blocked by the elastic body E, and a flow passage for leading a liquid in a container to the outside of the container is secured by the channel 68 and the passage 52.

The second bottom surface 62b of the first groove portion 24 is formed on the back side of the needle portion 22 with respect to the surface of the passage 52 on the back side. Thus, the distance between the bottom surface 62 and the protrusion 64 is set to be larger than the diameter of the passage 52. Therefore, even if the elastic body E around the puncture hole H enters between the pair of protrusions 64, a flow passage for a fluid such as a liquid is more reliably secured.

When the axial center C of the needle portion 22 is inserted perpendicularly into the outer surface Eo and the inner surface Ei of the elastic body E at an appropriate speed and the elastic body E around the puncture hole H is gradually pushed away, the occurrence of coring is prevented since there is no part to scrape off the elastic body E around the puncture hole H.

Then, the needle portion 22 of the puncture needle 14 is gradually inserted while the elastic deformation of the elastic body E is suppressed to the minimum by the protrusion ends 74 of the pair of protrusions 64 having an appropriate length in a direction along the axial center C and having a gentle change in the protruding length in a direction along the axial center C toward the outside of the intermediate portion 36 with respect to the bottom surface 62.

As shown in FIGS. 12A and 12B, the user of the puncture tool 10 further inserts the needle portion 22 into the puncture hole H of the elastic body E, and brings the distal surface of the connection portion 12 (the proximal end of the needle portion 22) into contact with or close to the outer surface Eo of the elastic body E. At this time, the closed end 66 of the first groove portion 24, for example, passes the inner surface Ei of the elastic body E and is inside the container. Alternatively, the closed end 66 of the first groove portion 24 may not pass through the inner surface Ei of the elastic body E. At this time, the protrusion ends 74 of the pair of protrusions 64 of the first groove portion 24 have already passed through the inner surface Ei of the elastic body E. Since the channel 68 of the first groove portion 24 is not blocked by the elastic body E and a flow passage for a liquid is secured, as described above, a liquid or gas can be put into a container through the channel 68 of the first groove portion 24 communicating with the passage 52. Also, the liquid in the container can be discharged to the outside of the container through the first groove portion 24 and the passage 52.

The distal ends 95 of the pair of protrusions 94 of the second groove portion 26 communicating with the other passage 54 are located further on the needle tip 34a side than the distal ends of the protrusion ends 74 of the pair of protrusions 64 of the first groove portion 24.

The user of the puncture tool 10 can then, for example, introduce air into the container or remove air from the container through the other passage 54.

An example of a method of putting a chemical solution into a container using a syringe will be briefly described. As the connector 8 shown in FIG. 1, a syringe is connected to the liquid passage 52, and an air bag (pressure adjuster) is connected to the gas passage 54. When a medicine or the like is moved from the syringe to the container through the passage 52 and the channel 68 of the first groove portion 24, the pressure in the container is increased, and the air in the container is moved to the air bag through the channel 98 of the second groove portion 26 and the passage 54. Thus, the pressure in the container is kept constant.

An example of a method of collecting a chemical solution in a container using a syringe will be briefly described. As the connector 8 shown in FIG. 1, a syringe is connected to the liquid passage 52, and an air bag is connected to the gas passage 54. The same amount of air as the amount of a chemical solution that is desired to be collected is put in the barrel of the syringe in advance, the piston of the syringe is pushed in, and the air in the barrel is injected into the container through the liquid passage 52. At this time, the pressure in the container increases, and the air in the container moves to the air bag through the gas flow passage 54, so that the pressure in the container is kept in equilibrium. Then, the container is arranged on the upper side and the syringe is arranged on the lower side.

For example, as shown in FIGS. 12A and 12B, when the user turns the container upside down in a state where the puncture needle 14 is inserted into the puncture hole H, the inner surface Ei of the elastic body E of the container is on the upper side and the outer surface Eo thereof is on the lower side. At this time, the elastic body E on the outer periphery of the puncture hole H of the elastic body E is in close contact with the outer peripheral surface of the body portion 32. In some cases, the elastic body E on the outer periphery of the puncture hole H of the elastic body E is in close contact with the outer peripheral surface of the intermediate portion 36. Thus, a fluid such as a liquid prevents a chemical solution, etc., from leaking from the gap between the outer peripheral surface of the body portion 32 and the puncture hole H of the elastic body E and the gap between the outer peripheral surface of the intermediate portion 36 and the puncture hole H of the elastic body E. Then, the liquid in the container gathers on the inner surface Ei of the elastic body E.

When the piston of the syringe is pulled back by the amount of a chemical solution desired to be collected, the liquid is introduced into the passage 52 through the channel 68 of the first groove portion 24. Thus, the chemical solution in the container moves to the barrel through the liquid passage 52, and the chemical solution is collected. At this time, the pressure in the container decreases, and the air in the air bag moves to the container through the gas flow passage 54, so that the pressure in the container is kept in equilibrium.

If the closed end 66 of the first groove portion 24 is arranged above the inner surface Ei of the elastic body E in a state where the user turns the container upside down, the closed end 66 of the first groove portion 24 is arranged on the outer surface Eo side of the elastic body E with respect to the inner surface Ei of the elastic body E, as shown in FIG. 13. At this time, the liquid is collected in the hole 52a through the channel 68 of the first groove portion 26.

After using the puncture needle 14 as appropriate, the user arranges the outer surface Eo of the elastic body E of the container on the upper side and the inner surface Ei thereof on the lower side before pulling out the puncture needle 14 from the puncture hole H of the elastic body E. In this state, the user pulls out the puncture needle 14 from the puncture hole H of the elastic body E. At this time, the elastic body E around the puncture hole H closes the puncture hole H while being in close contact with the outer peripheral surface of the puncture needle 14 with its elastic force. Thus, when the needle tip 34a is arranged closer to the outer surface Eo than the inner surface Ei of the elastic body E, the puncture hole H on the inner surface Ei of the elastic body E is closed before the needle tip 34a is pulled out from the outer surface Eo of the elastic body E. In this manner, the puncture hole H is gradually closed as the puncture needle 14 is pulled out from the elastic body E. Thus, foreign matter is prevented from entering the container from the outside of the container when the puncture needle 14 according to the present embodiment is pulled out from the elastic body E.

The puncture needle 14 according to the present embodiment demonstrates the following.

The first groove portion 24 forming the channel 68 communicating with the passage 52 of the body portion 32 is provided in the intermediate portion 36 of the needle portion 22 of the puncture needle 14 made of a synthetic resin. The first groove portion 24 includes the pair of protrusions 64 that extend from the body portion 32 toward the puncture portion 34 in a direction along the axial center C of the needle portion 22 and protrude from the bottom surface 62 in a direction intersecting the axial center C of the needle portion 22. The pair of protrusions 64 can function as ribs on the front side of the needle portion 22. Thus, the needle portion 22 is prevented from being unintentionally bent with respect to the axial center C, for example, when the needle portion 22 of the puncture needle 14 punctures the elastic body E or the like.

In addition, if the pair of protrusions 64 of the first groove portion 24 reaches the inner surface Ei of the elastic body E when the puncture needle 14 of the puncture tool 10 punctures the elastic body E, a part of the channel 68 can communicate with the inside of the container even if the position including the closed end 66 in the pair of protrusion ends 74 of the first groove portion 24 is blocked by the elastic body E. If the state in which the pair of protrusions 64 of the first groove portion 24 are in contact with the perforation of the elastic body E is maintained, the gap between the pair of protrusions 64 can be used as the channel 68 for a chemical solution. Thus, by turning a container such as a vial upside down and using the container with the inner surface Ei of the elastic body E facing upward and the outer surface Eo of the elastic body E facing downward, for example, it is possible to guide the liquid in the container to the channel 68 and prevent the liquid from remaining in the container. Therefore, the puncture needle 14 made of a resin material that facilitates securing of the passage 52 for a fluid when the elastic body E serving as a lid of a container enclosing a liquid such as a chemical solution is punctured, the puncture tool 10 including the puncture needle 14, and the puncture tool assembly 1 including the puncture tool 10 are provided.

Also, by forming the length of the pair of protrusions 64 of the first groove portion 24 along the axial center C of the needle portion 22 to be an appropriate length, it is possible to suppress the adjustment of the puncture depth of the puncture needle 14 with respect to the elastic body E having various thicknesses, and it is possible to suppress the leakage of the liquid from the inside of the container.

In addition, even if the elastic body E having various thicknesses is punctured, the pair of protrusions 64 extending from the body portion 32 toward the puncture portion 34 in a direction along the axial center C of the needle portion 22 can secure the channel 68 for a fluid, and the puncture needle 14 with improved stability of the puncture state can be provided.

A part of the protrusion ends 74 of the pair of protrusions 64 is formed as edges (parallel portions) 82 and 84 parallel to the axial center C (longitudinal direction) of the needle portion 22. When the edge (parallel portion) 82 of the protrusion end (opening edge) 74 of the needle portion 22 passes through the inner surface Ei of the puncture hole H of the elastic body E, the size of the puncture hole H may not change depending on the edge (parallel portion) 82, or even if the size changes, the amount of change is small. Thus, the edge (parallel portion) 82 can reduce the amount of change in the elastic deformation of the elastic body E around the puncture hole H. Therefore, when the puncture hole H is formed by the puncture needle 14, it is possible to increase the puncture amount of the puncture needle 14 while suppressing an increase in puncture resistance.

Likewise, when the edge (parallel portion) 84 of the protrusion end (opening edge) 74 of the needle portion 22 passes through the inner surface Ei of the puncture hole H of the elastic body E, the size of the puncture hole H may not change depending on the edge (parallel portion) 84, or even if the size changes, the amount of change is small. Thus, the edge (parallel portion) 84 can reduce the amount of change in the elastic deformation of the elastic body E around the puncture hole H. Therefore, when the puncture hole H is formed by the puncture needle 14, it is possible to increase the puncture amount of the puncture needle 14 while suppressing an increase in puncture resistance.

In this manner, it is possible to increase the puncture depth of the needle portion 22 of the puncture needle 14 with respect to the elastic body E while maintaining the puncture resistance constant when the puncture needle 14 made of a synthetic resin punctures the elastic body E of a container. Thus, the state in which the puncture needle 14 is inserted into the elastic body E can be maintained. For example, the elastic force of the elastic body E can prevent the puncture needle 14 from coming off from the elastic body E. Thus, with the edges (parallel portions) 82 and 84 of the protrusion end (opening edge) 74 of the needle portion 22, it is possible to improve the stability of the state in which the puncture needle 14 punctures the elastic body E.

The amount of protrusion of the edges 82 and 84 of the pair of protrusions 64 from the bottom surface 62 is larger on the side closer to the body portion 32 than on the side closer to the puncture portion 34. The amount of protrusion of the first edge 82 adjacent to the body portion 32 of the pair of protrusions 64 is larger than the amount of protrusion of the second edge 84 on the side closer to the puncture portion 34 than the first edge 82. Thus, when the puncture needle 14 is inserted into the puncture hole H of the elastic body E and the puncture depth is increased, the load (resistance) applied to the user's finger or the like can be reduced in a case where two different edges 82 and 84, whose heights with respect to the bottom surface 62 along the axial center C of the needle portion 22 are lower on the distal side and higher on the proximal end side are present in the elastic body E due to a puncture, as compared to a case where one edge 82 is present in the elastic body E along the axial center C of the needle portion 22 due to a puncture. In addition, forming the pair of protrusions 64 in this manner makes it less likely to form a gap between the puncture needle 14 and the puncture hole H when the puncture needle 14 punctures the elastic body E.

Forming, between the two edges 82 and 84, the inclined portion 86 that is inclined with respect to the axial center C of the needle portion 22 and has an amount of protrusion with respect to the bottom surface 62 which decreases from the edge 82 on the more proximal side toward the edge 84 on the more distal side allows the puncture needle 14 to be more smoothly inserted into the elastic body E.

The inclined portion 86 is arranged between the first edge 82 and the second edge 84 and has an amount of protrusion with respect to the bottom surface 62 which decreases from the first edge 82 toward the second edge 84. Thus, when the puncture needle 14 punctures the elastic body E, the first edge 82 and the second edge 84 can be smoothly connected to each other by the inclined portion 86. Forming the pair of protrusions 64 in this manner makes it less likely to form a gap between the puncture needle 14 and the puncture hole H when the puncture needle 14 punctures the elastic body E.

The bottom surface 62 of the first groove portion 24 is formed on the back side of the puncture needle 14 with respect to the inner peripheral surface of the passage 52 on the back side or in a position that coincides with the axial center C of the puncture needle 14. It is possible to secure a necessary depth of the first groove portion 24 while making the outer diameter (cross-sectional area) of the intermediate portion 36 of the puncture needle 14 relatively small. Thus, the first groove portion 24 can prevent the channel 68 as a fluid passage from being blocked when the puncture needle 14 punctures the elastic body E. Also, making the outer diameter (cross-sectional area) of the intermediate portion 36 of the puncture needle 14 relatively small can reduce the puncture resistance when the puncture needle 14 made of a synthetic resin punctures the elastic body E of the container.

The puncture needle 14 includes the first columnar portion 42 and the second columnar portion 44 of the body portion 32, the second columnar portion 44 is formed to have an outer diameter smaller than that of the first columnar portion 42, and the protrusion end (opening edge) 74 of the first groove portion 24 is formed closer to the distal side than the second columnar portion 44. Thus, the second columnar portion 44, which is inserted into the elastic body E earlier than the first columnar portion 42, is thinner, so that the puncture resistance when the puncture needle 14 made of a synthetic resin punctures a medicine container is minimized, and the sealing performance near the boundary between the second columnar portion 44 and the first columnar portion 42 or the sealing performance between the first columnar portion 42 and the elastic body E is improved, so that it is possible to prevent a chemical solution from leaking from the gap between the puncture needle 14 and the elastic body E due to a change in the internal pressure in the container occurring when a medicine is injected or collected, or to prevent a substance outside the container from unintentionally mixing into the liquid in the container.

Also, with the puncture portion 34 having a substantially conical shape, the puncture portion 34 is less likely to be crushed by the resistance of the elastic body E when the needle tip 34a of the puncture needle 14 is inserted into the elastic body E. Thus, it is possible to reduce an increase in puncture resistance caused by the crushing of the puncture portion 34.

The axial center C of the first columnar portion 42, the axial center of the second columnar portion 44, and the axial center including the needle tip 34a of the puncture portion 34 are deviated from each other. Thus, the channel 68 and the passage 52 of the first groove portion 24 can be secured even at a position shifted from the axial center C of the needle portion 22. Also, the pointed needle tip 34a can be formed without the channel 68 of the first groove portion 24 being arranged in the needle tip 34a. Thus, when the puncture needle 14 punctures the elastic body E, more reliable puncturing can be achieved.

The cross-sectional area of the channel 68 perpendicular to the axial center C of the needle portion 22 is equal to or greater than the cross-sectional area of the passage 52 perpendicular to the axial center C of the needle portion 22. Thus, more fluid can be collected in the channel 68, for example, in the container.

The distance between the edges 82 and 84 of the pair of protrusions 64 protruding from the bottom surface 62 is the same as the width of the bottom surface 62 of the first groove portion 24. Thus, forming the opening on the front side, which serves as an inlet for a liquid or the like to the channel 68, to have an appropriate size can collect a larger amount of fluid in the channel 68, for example, in the container.

The length of the pair of protrusions 64 of the first groove portion 24 along the axial center C of the needle portion 22 is formed to be equal to or larger than the length of penetration into the inner surface Ei of the plate-shaped elastic body E punctured from the outer surface Eo side. Thus, when the elastic body E is punctured with the puncture needle 14, the distal end of the first groove portion 24 can be arranged in the container in a state where the needle tip 34a has passed the outer surface Eo and the inner surface Ei of the elastic body E in this order and the closed end 66 at the proximal end of the first groove portion 24 has passed the outer surface Eo of the elastic body E. As such, fluid can be collected in the channel 68 in the container.

In this manner, with an appropriate relationship between the depth of the first groove portion 24 and the width between the protrusion ends (opening edges) 74 of the first groove portion 24 and the width between the wall surfaces 72 of the first groove portion 24, even if the puncture needle 14 punctures an elastic body E having different thicknesses and/or hardnesses, for example, an elastic body E having a low hardness, being easily deformed, and having a large thickness, the elastic body E does not block the channel 68 between the pair of protrusions 64 of the first groove portion 24, and the fluid passage 52 can be secured. Thus, even if the puncture needle 14 punctures an elastic body E having different thicknesses, a chemical solution in the container can be collected to the end due to the presence of the first groove portion 24 of the passage 52 of the puncture needle 14.

Since the pair of protrusions 94 of the second groove portion 26 function as ribs on the back side, it is possible to more reliably prevent the buckling of the needle portion 22 together with the pair of protrusions 64 of the first groove portion 24.

The distal ends 95 of the protrusions 94 of the second groove portion 26 communicating with the passage 54 are located closer to the needle tip 34a than the distal ends 65 of the pair of protrusions 64 of the first groove portion 24. Thus, when the container is turned upside down in a state where the puncture needle 14 punctures the elastic body E, the distal ends 95 of the protrusions 94 of the second groove portion 26 are arranged above the distal ends 65 of the pair of protrusions 64 of the first groove portion 24. When air is introduced into the container through the passage 54 in this state, for example, the chemical solution in the container can be efficiently taken out through the passage 52.

### (Modifications)

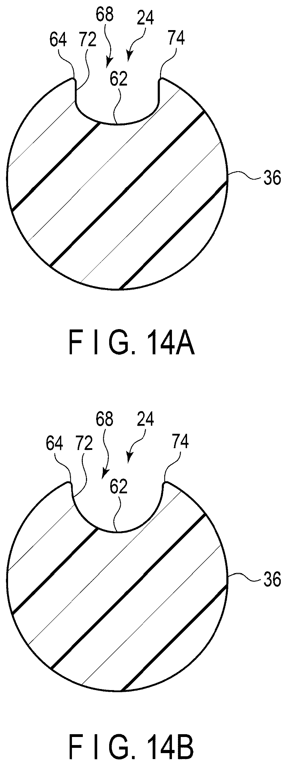

Here, a modification of the shape of the channel 68 of the first groove portion 24 of the needle portion 22 described in the first embodiment will be described with reference to FIGS. 14A to 14E.

FIGS. 14A to 14E schematically show a modification of the cross-sectional view taken along the line IX-IX in FIG. 4. The passage 54 is not shown in FIGS. 14A to 14E.

In the foregoing first embodiment, an example in which the wall surfaces 72 of the pair of protrusions 64 of the first groove portion 24 are parallel to each other and the height of the bottom surface 62 is constant along the left-right direction of the needle portion 22, as shown in FIG. 9, has been described.

As shown in FIG. 14A, the bottom surface 62 may be formed in a concave shape such as a substantially half pipe shape that is convex toward the back side along the left-right direction. Thus, the wall surfaces 72 and the bottom surface 62 of the pair of protrusions 64 form a channel 68 having a substantially U-shaped cross section orthogonal to the axial center C of the needle portion 22 of the puncture needle 14. The bottommost portion of the second bottom surface 62b may be located in a position along the axial center C of the needle portion 22, or located on the front side or the back side of the axial center C of the needle portion 22.

As shown in FIG. 14B, the wall surfaces 72 of the pair of protrusions 64 of the first groove portion 24 may not be parallel to each other. In the example shown in FIG. 14B, the wall surfaces 72 and the bottom surface 62 of the pair of protrusions 64 form a channel 68 having a cross section in a concave shape such as a substantially U-shape or a substantially D-shape orthogonal to the axial center C of the needle portion 22 of the puncture needle 14. Manufacturing is facilitated if the width between the wall surfaces 72 of the first groove portion 24 is equal to or less than the width between the edges (opening surfaces) 82, which depends on a mold used. The puncture needle 14 is preferably manufactured by, for example, injection-molding, but may be manufactured by, for example, using a 3D printer.

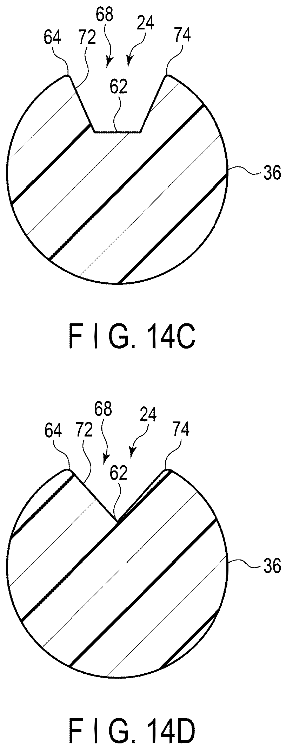

As shown in FIG. 14C, the height of the bottom surface 62 is constant in the left-right direction of the needle portion 22, but the wall surfaces 72 of the pair of protrusions 64 of the first groove portion 24 may not be parallel to each other. In the example shown in FIG. 14C, the wall surfaces 72 and the bottom surface 62 of the pair of protrusions 64 form a channel 68 having a cross section in a concave shape such as a substantially trapezoidal shape orthogonal to the axial center C of the needle portion 22 of the puncture needle 14. The distance between the protrusion ends (opening edges) 74 of the pair of protrusions 64 protruding from the bottom surface 62 is larger than the width of the bottom surface 62 of the first groove portion 24.

As shown in FIG. 14D, the boundary between the wall surfaces 72 and the bottom surface 62 is preferably ambiguous. In this case, the bottommost portion of the bottom surface 62 is formed in a linear shape along the axial center C of the needle portion 22. In the example shown in FIG. 14D, the wall surfaces 72 and the bottom surface 62 of the pair of protrusions 64 form a channel 68 having a cross section in a concave shape such as a substantially triangular shape orthogonal to the axial center C of the needle portion 22 of the puncture needle 14.

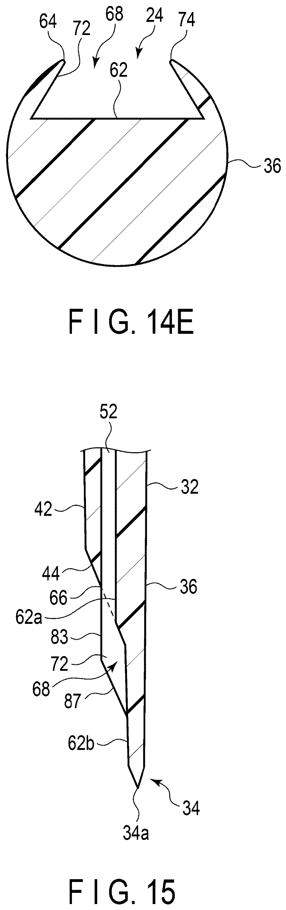

As shown in FIG. 14E, the width of the bottom surface 62 in the left-right direction may be larger than the width of the protrusion end (opening edge) 74 in the left-right direction. In the channel 68 shown in FIG. 14C, the shorter one of the bases of the trapezoid is on the bottom surface 62, and in the channel 68 shown in FIG. 14E, the longer one of the bases of the trapezoid is on the bottom surface 62. In the example shown in FIG. 14E, the width of the protrusion ends (opening edges) 74 in the left-right direction (the shorter one of the bases of the trapezoid) may be any size as long as it allows a liquid to flow toward the bottom surface 62 of the first groove portion 24. In the example shown in FIG. 14E, the wall surfaces 72 and the bottom surface 62 of the pair of protrusions 64 form a channel 68 having a cross section in a concave shape such as a substantially trapezoidal shape orthogonal to the axial center C of the needle portion 22 of the puncture needle 14. The distance between the protrusion ends 74 of the pair of protrusions 64 protruding from the bottom surface 62 is smaller than the width of the bottom surface 62 of the first groove portion 24. Such a puncture needle 14 may be manufactured using, for example, a 3D printer.

In this manner, the channel 68 of the first groove portion 24 is formed in various shapes. As explained in the first embodiment, the shape of the protrusion ends 74 of the pair of protrusions 64 may be formed such that the body portion 32 side is a substantially linear edge and the puncture portion 34 side is an edge wider than the edge 82 side. Alternatively, the shape of the protrusion ends 74 of the pair of protrusions 64 may be formed such that the entirety of the protrusion ends 74 are in a linear shape.

Although not shown, the wall surfaces 72 of the pair of protrusions 64 may be parallel to each other toward the opposing wall surfaces 72 side, or may protrude in a direction in which the wall surfaces 72 approach each other or be recessed in a direction in which the wall surfaces 72 are separated from each other as long as the channel 68 can be secured. That is, the wall surfaces 72 of the pair of protrusions 64 may be flat surfaces, appropriately curved surfaces, or the like.

### (Second Embodiment)

The needle portion 22 of the puncture needle 14 of the puncture tool 10 according to the second embodiment will be described with reference to FIG. 15. The present embodiment is a modification of the first embodiment including each modification example, and the same members as the members described in the first embodiment or members having the same functions as those of the members described in the first embodiment are denoted by the same reference numerals to the extent possible, and detailed description thereof will be omitted.

FIG. 15 schematically shows a modification of the cross section of the puncture needle 14 of the puncture tool 10 shown in FIGS. 4 and 5 taken along the line VIII-VIII.

The connection portion 12 and the puncture needle 14 of the puncture tool 10 according to the first embodiment have been described as having two fluid passages 52 and 54. The connection portion 12 and the puncture needle 14 of the puncture tool 10 according to the present embodiment have only one fluid passage 52. In the puncture needle 14 according to the first embodiment, the first groove portion 24 is arranged on the front side and the second groove portion 24 is arranged on the back side. In the present embodiment, the first groove portion 24 is arranged on the front side, but the second groove portion 26 is not arranged on the back side. In the present embodiment, the passage 52 and the channel 68 are used as passages for both liquid and gas. For example, a syringe is connected as the connector 8 of the puncture tool 10 having the puncture needle 14 according to the present embodiment.

The protrusion ends 74 of the pair of protrusions 64 of the first groove portion 24 are formed as one edge (parallel portion) 83 different from the two edges 82 and 84 (see FIGS. 6 and 8) along the axial center C of the needle portion 22 and one inclined portion 87 different from the two inclined portions 86 and 88 (see FIGS. 6 and 8) along the axial center C of the needle portion 22.

The first bottom surface 62a is arranged along the inner peripheral surface of the passage 52 on the back side. The second bottom surface 62b is not arranged in a position along the inner peripheral surface of the passage 52 on the back side, but is arranged on the back side opposite to the opening side (front side) of the first groove portion 24 with respect to the inner peripheral surface on the flat surface side of the passage 52.

The puncture needle 14 may be formed in this manner. When the body portion 32, the puncture portion 34, and the intermediate portion 36 of the needle portion 22 of the puncture needle 14 according to the present embodiment have the same dimensions as those of the needle portion 22 described in the first embodiment, the length of the edge 83 can be formed to be approximately the length obtained by combining the edges 82 and 84. Thus, when the puncture needle 14 punctures the elastic body E, the channel 68 can be secured, and the punctured state in which the needle portion 22 is inserted into the puncture hole H can be stably maintained.

Therefore, the bottom surface 62 may be arranged in a position along the inner peripheral surface of the passage 52 on the back side and/or in a position on the back side opposite to the opening side of the first groove portion 24 with respect to the inner peripheral surface of the passage 52 on the back side.

### (First Modification)

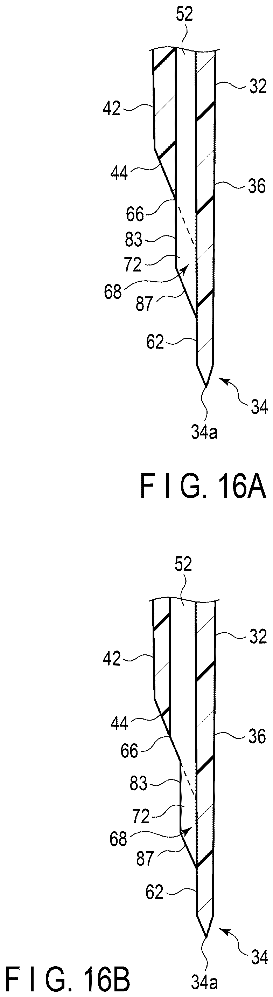

A first modification of the puncture needle 14 of the puncture tool 10 according to the second embodiment will be described with reference to FIG. 16A. FIG. 16A shows a first modification of the cross-sectional view of the needle portion shown in FIGS. 4 and 5 taken along the line VIII-VIII.

As shown in FIG. 16A, the bottom surface 62 is formed straight in parallel to the axial center C of the needle portion 22. Thus, the bottom surface 62 is formed, for example, as a single surface. The bottom surface 62 is connected straight to the inner peripheral surface of the passage 52 on the back side. Thus, the passage 52 and the channel 68 of the first groove portion 24 are in direct communication with each other. The bottom surface 62 may be a flat surface or a curved surface.

The protrusion ends 74 of the pair of protrusions 64 of the first groove portion 24 are formed as one edge (parallel portion) 83 along the axial center C of the needle portion 22 and one inclined portion 87 along the axial center C of the needle portion 22.

The puncture needle 14 may be formed in this manner.

### (Second Modification)

A second modification of the puncture needle 14 of the puncture tool 10 according to the second embodiment will be described with reference to FIG. 16B. The puncture needle 14 according to the present modification is a further modification of the first modification. FIG. 16B shows the second modification of the cross-sectional view of the needle portion shown in FIGS. 4 and 5 taken along the line VIII-VIII.

As shown in FIG. 16B, the closed end 66 of the first groove portion 24 is arranged in the second columnar portion 44 of the body portion 32, not in the first intermediate body 36a of the intermediate portion 36. That is, the first groove portion 24 is preferably arranged not only in the intermediate portion 36 and the puncture portion 34 but also in the body portion 32.

The puncture needle 14 may be formed in this manner.

### (Third Modification)

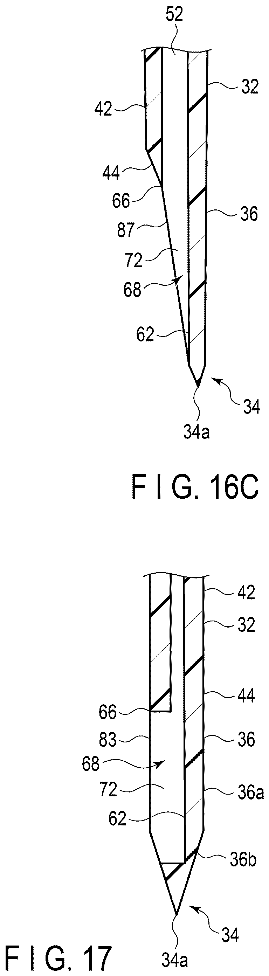

A third modification of the puncture needle 14 of the puncture tool 10 according to the second embodiment will be described with reference to FIG. 16C. The puncture needle 14 according to the present modification is a further modification of the first modification or the second modification. FIG. 16C shows the third modification of the cross-sectional view of the needle portion shown in FIGS. 4 and 5 taken along the line VIII-VIII.

As shown in FIG. 16C, the protrusion ends 74 of the pair of protrusions 64 of the first groove portion 24 are formed as one inclined portion 87 along the axial center C of the needle portion 22. Even if the puncture needle 14 is inserted into the elastic body E, the channel 68 is secured.

The puncture needle 14 may be formed in this manner.

### (Third Embodiment)

The puncture needle 14 of the puncture tool 10 according to a third embodiment will be described with reference to FIG. 17.

As shown in FIG. 17, the protrusion ends 74 of the pair of protrusions 64 of the first groove portion 24 may be formed along the outer peripheral surface of the intermediate portion 36 between the distal end of the body portion 32 and the proximal end of the puncture portion 34. The protrusion ends 74 of the pair of protrusions 64 of the first groove portion 24 are formed as one edge (parallel portion) 83 along the axial center C of the needle portion 22.

For example, the central axis (axial center) of the second columnar portion 44 of the body portion 32 and the axial center of the needle tip 34a of the puncture portion 34 coincide with each other. Also, if the outer diameters of the body portion 32 and the first intermediate body 36a of the intermediate portion 36 have the same shape up to the proximal end of the second intermediate body 36b, the axial center C of the needle portion 22 can pass through the needle tip 34a of the puncture portion 34 in the puncture needle 14 according to the present embodiment.

The puncture needle 14 may be formed in this manner.

Therefore, according to the first to third embodiments, it is possible to provide a puncture needle made of a resin material that facilitates securing of a fluid passage when an elastic body serving as a lid of a container enclosing a liquid such as a chemical solution is punctured, a puncture tool including the puncture needle, and a puncture tool assembly including the puncture tool.

The invention of the present application is not limited to the above-described embodiment, and can be variously modified at the implementation stage without departing from the gist thereof. In addition, each embodiment may be carried out in combination as appropriate as possible, and in that case, the combined effect can be obtained. Further, the above-described embodiment includes inventions at various stages, and various inventions can be extracted by an appropriate combination in a plurality of disclosed constitutional requirements. For example, if the problem can be solved and an effect can be obtained even if some elements are deleted from all the elements described in the embodiment, the element from which these elements are deleted can be extracted as an invention.

### Reference Signs List

1 ... Puncture tool assembly, 8 ... Connector, 10 ... Puncture tool, 12 ... Connection portion, 14 ... Puncture needle, 22 ... Needle portion, 24 ... First groove portion, 26 ... Second groove portion, 32 ... Body portion, 34 ... Puncture portion, 34a ... Needle tip, 36 ... Intermediate portion, 36a ... First intermediate body, 36b ... Second intermediate body, 42 ... First columnar portion, 44 ... Second columnar portion, 44a ... Inclined portion, 52 ... Passage, 52a ... Hole, 54 ... Passage, 54a ... Hole, 62 ... Bottom surface, 62a ... First bottom surface (flat surface), 62b ... Second bottom surface (flat surface), 62c ... Third bottom surface (inclined surface), 63 ... Distal end, 64 ... Protrusion, 65 ... Distal end, 66 ... Closed end, 68 ... Channel (flow passage), 72 ... Wall surface, 74 ... Protrusion end, 82 ... First edge (opening edge), 84 ... Second edge (opening edge), 86 ... First inclined portion (opening edge), 88 ... Second inclined portion (opening edge), 92 ... Bottom surface, 94 ... Protrusion, 94a ... Closed end, 94b ... Edge, 95 ... Distal end, 98 ... Channel, E ... Elastic body, Ei ... inner surface, Eo ... Outer surface, H ... Puncture hole.

## Claims

1. A puncture needle made of a resin material, the puncture needle comprising:
a needle portion which integrally includes:
a body portion including a passage configured to pass a fluid through the passage,
a puncture portion arranged on a distal side of the body portion and including a needle tip at a most distal end, and
an intermediate portion between the body portion and the puncture portion; and
a groove portion which includes:
a bottom surface integrally arranged on the intermediate portion and extending from the body portion toward the puncture portion, and
a pair of protrusions integrally arranged on the bottom surface, extending from the body portion toward the puncture portion in a direction along an axial center of the needle portion, and protruding from the bottom surface in a direction intersecting the axial center of the needle portion,
wherein a channel formed between the bottom surface and the pair of protrusions communicates with the passage of the body portion, and the channel is opened toward an outside of the intermediate portion.

2. The puncture needle according to claim 1, wherein:
at least a part of an edge of each of the pair of protrusions protruding from the bottom surface is parallel to a direction along the axial center of the needle portion.

3. The puncture needle according to claim 2, wherein:
the edges of the pair of protrusions include:
a first edge arranged adjacent to the body portion and protruding from the bottom surface, and
a second edge arranged on a side closer to the puncture portion than the first edge and protruding from the bottom surface, and
an amount of protrusion of the first edge is larger than an amount of protrusion of the second edge with respect to the bottom surface.

4. The puncture needle according to claim 3, wherein:
the pair of protrusions includes an inclined portion arranged between the first edge and the second edge,
the inclined portion being inclined with respect to the axial center of the needle portion, and having an amount of protrusion with respect to the bottom surface which decreases as the inclined portion extends from the first edge toward the second edge.

5. The puncture needle according to claim 1 or 2, wherein:
the bottom surface is arranged in a position along an inner peripheral surface of the passage on a side opposite to an opening side of the groove portion, or is arranged on a side opposite to the opening side of the groove portion with respect to the inner peripheral surface of the passage.

6. The puncture needle according to claim 1 or 2, wherein:
the body portion includes:
a first columnar portion including the passage; and
a second columnar portion arranged between the first columnar portion and the puncture portion and including an opening edge of the passage, wherein
the second columnar portion has an outer diameter equal to or smaller than an outer diameter of the first columnar portion, and
an outer peripheral surface of the second columnar portion is continuous with an outer peripheral surface of the puncture portion.

7. The puncture needle according to claim 6, wherein:
an axial center of the first columnar portion, an axial center of the second columnar portion, and an axial center including the needle tip of the puncture portion are deviated from each other.

8. The puncture needle according to claim 1 or 2, wherein:
a cross-sectional area of the channel orthogonal to the axial center of the needle portion is equal to or larger than a cross-sectional area of the passage orthogonal to the axial center of the needle portion.

9. The puncture needle according to claim 1 or 2, wherein:
a distance between the edges of the pair of protrusions protruding from the bottom surface is equal to or larger than a width of the bottom surface of the groove portion.

10. The puncture needle according to claim 1 or 2, wherein:
a length of the pair of protrusions of the groove portion along the axial center of the needle portion is formed to be equal to or larger than a length of penetration into an inner surface of a plate-shaped elastic body punctured from an outer surface side.

11. A puncture tool which integrally comprises:
the puncture needle according to claim 1 or 2; and
a base arranged with the body portion of the puncture needle.

12. A puncture tool assembly comprising:
the puncture tool according to claim 11; and
a connector connected to the base of the puncture tool.
